# EUROPEAN PATENT APPLICATION

(11) **EP 4 696 678 A1**
(43) Date of publication of application: **18.02.2026**
(21) Application number: 24788593.2
(22) Date of filing: 29.03.2024
(51) Int. Cl.: C07C 319/02, C07C 323/52, C07B 61/00, C07C 327/32

(54) **METHOD FOR PRODUCING 3-MERCAPTOCARBOXYLIC ACID**

(30) Priority: 13.04.2023 JP 2023065708
(71) Applicant: Resonac Corporation, Tokyo 105-7325 (JP)
(72) Inventor: NAKAMURA, Shinya, Tokyo 105-7325 (JP)
(74) Representative: Strehl & Partner mbB
(86) International application number: PCT/JP2024/012967
(87) International publication number: WO 2024/214565

(57) **Abstract**

The present invention provides a method for producing 3-mercaptocarboxylic acid under industrially advantageous conditions.

The present invention includes a method for producing a 3-mercaptocarboxylic acid represented by formula (1), the method including the following step (A) and step (B):
step (A):
a step of adding a thiocarboxylic acid to the carbon-carbon unsaturated bond of an α,β-unsaturated carboxylic acid to obtain a thiocarboxylic acid ester; and
step (B):
adding water and an acid catalyst to the thiocarboxylic acid ester for a hydrolysis reaction, wherein the hydrolysis reaction is under a condition where the amount of the acid catalyst used in the reaction is from 0.025 equivalents to 0.68 equivalents relative to the thiocarboxylic acid ester, and the amount of water is 1.0 equivalent or more relative to the thiocarboxylic acid ester, 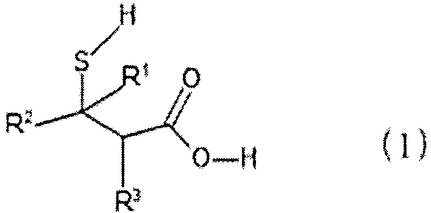 wherein R¹ to R³ each independently represent a hydrogen atom or an alkyl group having 1 to 5 carbon atoms.

## Description

### Technical Field

The present invention relates to a method for producing 3-mercaptocarboxylic acid.

### Background Art

A compound having both a mercapto group and a carboxyl group can form an ester structure by the reaction of its carboxyl group with a hydroxyl group. By using a polyhydric alcohol, as a hydroxyl group-containing compound, a compound having a plurality of mercapto groups can be obtained. The polyvalent thiol compound thus obtained is useful as a raw material for resins, thermosetting resins, and photocurable resins, by utilizing the fact that the mercapto groups readily react with such as epoxy groups and unsaturated double bonds, and therefore possesses high industrial value.

Accordingly, there has been a demand for the establishment of an efficient method for producing compounds having both a mercapto group and a carboxyl group.

As methods for synthesizing compounds having both a mercapto group and a carboxyl group, various methods are known, such as: the addition reaction of sodium sulfide with α,β-unsaturated carboxylic acids, the reaction of sodium sulfide with β-halocarboxylic acids, the addition reaction of sodium sulfide with α,β-unsaturated carboxylic acids, the reaction of sodium sulfide with β-halocarboxylic acids, the addition reaction of thiourea with α,β-unsaturated carboxylic acids followed by hydrolysis, and the addition reaction of thiocarboxylic acids with α,β-unsaturated carboxylic acids followed by hydrolysis.

The method in which α,β-unsaturated carboxylic acids and thiocarboxylic acids are used as raw materials is disclosed in Non-Patent Literatures 1 to 3. Non-Patent Literature 1 discloses an example of the synthesis of 3-mercaptobutyric acid, in which the hydrolysis of the intermediate thioester is carried out under alkaline conditions. In this case, it is necessary to neutralize the resulting alkali salt of the product and then perform extraction, which increases the cost and is therefore not suitable as an industrial production method. Non-Patent Literatures 2 and 3 describe examples of the synthesis of 2-mercaptomethylbutyric acid. In both cases, the intermediate thioester is hydrolyzed with acid; however, the yield cannot be considered sufficient.

### Citation List

### Non Patent Literature

[Non Patent Literature 1]
   Biomacromolecules 2001, 2, 1061-1065.
[Non Patent Literature 2]
   Med. Chem. 11(2003), 4685-4691.
[Non Patent Literature 3]
   Biochemistry 2007, 56, 6087-6097.

### Summary of Invention

### Technical Problem

Provided is a method for producing 3-mercaptocarboxylic acid under industrially advantageous conditions.

### Solution to Problem

The present inventors have conducted extensive studies to solve the above-mentioned problems, and as a result, have completed the present invention.

That is, the present invention relates to the methods for producing 3-mercaptocarboxylic acid following [1] to [7].
[1] A method for producing a 3-mercaptocarboxylic acid represented by formula (1), the method including the following step (A) and step (B):
   step (A):
      adding a thiocarboxylic acid to the carbon-carbon unsaturated bond of an α,β-unsaturated carboxylic acid to obtain a thiocarboxylic acid ester; and
   step (B):
      adding water and an acid catalyst to the thiocarboxylic acid ester for a hydrolysis reaction, wherein the hydrolysis reaction is under a condition where the amount of the acid catalyst used in the reaction is from 0.025 equivalents to 0.68 equivalents relative to the thiocarboxylic acid ester, and the amount of water is 1.0 equivalent or more relative to the thiocarboxylic acid ester, 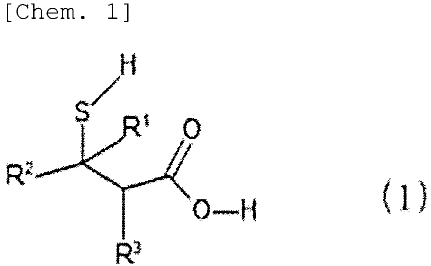 wherein R¹ to R³ each independently represent a hydrogen atom or an alkyl group having 1 to 5 carbon atoms.
[2] The method for producing 3-mercaptocarboxylic acid according to [1], in which, in the step (B), the amount of water is from 1.0 equivalent to 5.5 equivalents relative to the thiocarboxylic acid ester.
[3] The method for producing 3-mercaptocarboxylic acid according to [1] or [2], in which the addition reaction in the step (A) is carried out in the presence of a solvent, and after the reaction, the solvent is distilled off, followed by the step (B).
[4] The method for producing 3-mercaptocarboxylic acid according to any one of [1] to [3], in which the acid catalyst in the step (B) is hydrochloric acid or sulfuric acid.
[5] The method for producing 3-mercaptocarboxylic acid according to any one of [1] to [4], in which the thiocarboxylic acid is thioacetic acid.
[6] The method for producing 3-mercaptocarboxylic acid according to any one of [1] to [5], in which the α,β-unsaturated carboxylic acid is crotonic acid.
[7] The method for producing 3-mercaptocarboxylic acid according to [1] or [2], in which the acid catalyst is sulfuric acid, the thiocarboxylic acid is thioacetic acid, the α,β-unsaturated carboxylic acid is crotonic acid, and the 3-mercaptocarboxylic acid represented by formula (1) is 3-mercaptobutyric acid.

### Advantageous Effects of Invention

According to the present invention, 3-mercaptocarboxylic acid can be produced under industrially advantageous conditions.

### Description of Embodiment

In the present specification, when a numerical range is expressed using "to," the numerical values at both ends are intended to be included as the upper and lower limits of the range.

In one embodiment of the present invention, the target 3-mercaptocarboxylic acid is represented by formula (1): 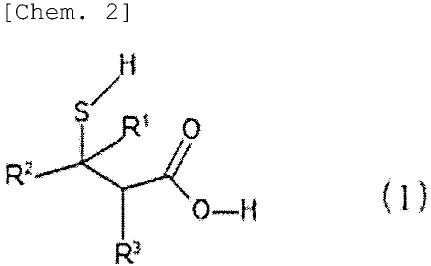 wherein R¹ to R³ each independently represent a hydrogen atom or an alkyl group having 1 to 5 carbon atoms.

In formula (1), R¹ to R³ each independently represent a hydrogen atom or an alkyl group having 1 to 5 carbon atoms. R¹ and R² are preferably each independently a hydrogen atom or an alkyl group having 1 to 3 carbon atoms. In addition, it is preferable that one of R¹ and R² is a hydrogen atom. It is also preferable that R³ is a hydrogen atom or an alkyl group having 1 to 3 carbon atoms, and more preferably a hydrogen atom or a methyl group.

Examples of the 3-mercaptocarboxylic acids represented by formula (1) include 3-mercaptopropionic acid, 3-mercaptoisobutyric acid, 3-mercaptobutyric acid, 3-mercapto-2-methylbutyric acid, 3-mercapto-3-methylbutyric acid, 3-mercaptovaleric acid, 3-mercapto-2-methylvaleric acid, 3-mercapto-4-methylvaleric acid, and 3-mercaptohexanoic acid. Among these, from the viewpoint of the balance between reactivity and pot life, 3-mercaptobutyric acid is preferred.

### <Step A>

In step A, a thiocarboxylic acid is subjected to an addition reaction with the carbon-carbon unsaturated bond of an α,β-unsaturated carboxylic acid.

As the α,β-unsaturated carboxylic acid used as a raw material, an α,β-unsaturated carboxylic acid corresponding to the target 3-mercaptocarboxylic acid represented by formula (1) is selected. Specifically, examples include acrylic acid, methacrylic acid, crotonic acid, angelic acid, tiglic acid, 3-methylcrotonic acid, 2-pentenoic acid, 2-methyl-2-pentenoic acid, 4-methyl-2-pentenoic acid, and 2-hexenoic acid. Among these, crotonic acid is preferred.

Examples of the thiocarboxylic acid include thioacetic acid, thiopropionic acid, and thiobenzoic acid. Among these, thioacetic acid is preferred.

The addition reaction between an α,β-unsaturated carboxylic acid and a thiocarboxylic acid is generally carried out either in a solvent or under solvent-free conditions. The reaction does not require any catalyst.

When a solvent is used, there is no particular limitation as long as the solvent does not inhibit the reaction between the α,β-unsaturated carboxylic acid and the thiocarboxylic acid. Specific examples of solvents include aromatic hydrocarbon solvents such as toluene and xylene, and aliphatic hydrocarbon solvents such as hexane, heptane, octane, and cyclohexane. Among these, toluene is preferred. There is no particular limitation on the amount of solvent used, but it is preferably 70 mass% or less based on the total reaction solution, and more preferably from 10 mass% to 50 mass%.

In step (A), a thiocarboxylic acid ester is obtained as an intermediate product. The thiocarboxylic acid ester is the α,β-unsaturated carboxylic acid with a hydrogen atom added at the α-position and a thiocarboxylic acid ester group formed at the β-position.

The reaction temperature is preferably from 50°C to 100°C. More preferably, from 60°C to 90°C.

The reaction time is not particularly limited as long as the reaction is complete. Preferably from 2 to 8 hours.

It is preferable to use the thiocarboxylic acid in excess amounts of equivalents relative to the unsaturated bond of the α,β-unsaturated carboxylic acid. Preferably, the thiocarboxylic acid is used in an amount of 1.1 to 1.5 equivalents, more preferably 1.2 to 1.3 equivalents, relative to the unsaturated bond of the α,β-unsaturated carboxylic acid. When used within this range, most of the α,β-unsaturated carboxylic acid reacts, so it is sufficient to simply remove the excess unreacted thiocarboxylic acid and the solvent. The reaction product from which the thiocarboxylic acid and solvent have been removed can be directly subjected to step (B).

### <Step (B)>

Step (B) is a step of reacting the thiocarboxylic acid ester obtained in step (A) with water in the presence of an acid catalyst for hydrolysis. In step (B), since water serves as the solvent, it is not necessary to use an additional solvent. When a solvent is used, there is no particular limitation as long as it does not inhibit the hydrolysis reaction. Aromatic hydrocarbon solvents such as toluene and xylene can be used.

Examples of acid catalysts include hydrochloric acid, sulfuric acid, nitric acid, and phosphoric acid. Among these, hydrochloric acid and sulfuric acid are preferred. With respect to corrosion resistance, particularly the minimization of corrosion of pipes and pumps by trace amounts of residual acid during distillation, sulfuric acid is more preferable. In contrast, from the perspective of stabilizing the contents with trace amounts of residual acid after distillation, hydrochloric acid is preferable.

It is preferable to use distilled water or ion-exchanged water as the water.

The temperature for the hydrolysis reaction is not particularly limited as long as the reaction proceeds, but is preferably from 60°C to 110°C. The reaction time is also not limited as long as the hydrolysis reaction is complete, but is preferably from 2 to 10 hours.

The amount of acid catalyst used in the hydrolysis reaction is from 0.025 equivalents to 0.68 equivalents relative to the thiocarboxylic acid ester. The equivalent of the acid catalyst refers to the equivalent relative to the thiocarboxylic acid attached via an ester bond to the α,β-unsaturated carboxylic acid at the β-position. When the equivalent of the acid catalyst is less than 0.025 or more than 0.68, the yield of 3-mercaptocarboxylic acid decreases, and therefore such conditions are not preferable. The equivalent of the acid catalyst is more preferably from 0.05 equivalents to 0.50 equivalents.

The amount of water used in the hydrolysis reaction is not particularly limited as long as it is 1.0 equivalent or more relative to the thiocarboxylic acid ester, but is preferably from 1.0 equivalent to 5.5 equivalents, and more preferably from 1.5 equivalents to 5.0 equivalents. Within these ranges, there is no decrease in the yield of 3-mercaptocarboxylic acid.

After the hydrolysis reaction, the resulting 3-mercaptocarboxylic acid can be extracted and isolated using an organic solvent such as toluene. Alternatively, the 3-mercaptocarboxylic acid can also be isolated by methods such as column chromatography or distillation.

### Examples

Hereinafter, the present invention will be described in detail with reference to examples and comparative examples, but the present invention is not intended to be limited to following examples.

### [Example 1]

### Step (A)

A three-neck flask equipped with a stirrer was charged with 100.0 g (1.16 mol) of crotonic acid, 110.5 g (1.45 mol) of thioacetic acid (CH₃(C=O)SH), and 50 mL of toluene as a solvent, and the resultant was allowed to react at 80 to 85°C for 6 hours. Subsequently, the reaction mixture was subjected to reduced pressure (3 kPa) using a diaphragm pump and heated at a bath temperature of 40°C to remove toluene and unreacted thioacetic acid by distillation under reduced pressure. The unreacted crotonic acid was confirmed to be less than 1% by ¹H-NMR analysis. Accordingly, the yield of the intermediate thiocarboxylic acid ester ((3-acetylthio)butyric acid) was regarded as 100%.

### Step (B)

The flask containing the thiocarboxylic acid ester as the reaction product obtained in step (A) was charged with concentrated hydrochloric acid and distilled water in the amounts shown in Table 1, and the resultant was subjected to a hydrolysis reaction at 100°C for 3 hours. The equivalent of hydrogen chloride relative to the thiocarboxylic acid ester was 0.50. Since the concentrated hydrochloric acid used was a 35 mass% aqueous solution, the equivalent of acid was calculated based on the number of moles of hydrogen chloride added. The water content from the concentrated hydrochloric acid as a solvent was included together with the separately added water when calculating the total equivalent of water relative to the thiocarboxylic acid ester. The progress of the hydrolysis reaction was tracked by ¹H-NMR, and the reaction was considered complete when the reaction reached 99% or more. Next, the reaction mixture was extracted with toluene in the amount shown in Table 1. The organic phase (toluene layer) was then washed with distilled water, using the amount and number of washes indicated in Table 1. The organic phase was concentrated by reducing the pressure to 3 kPa with a diaphragm pump and heating at a bath temperature of 40 to 60°C to remove toluene and acetic acid by distillation. Subsequently, the residue was distilled under reduced pressure at 0.5 kPa using a vacuum pump, and 3-mercaptobutyric acid was collected as the distillate. The distillation temperature was 60 to 70°C. The results (yield amount, yield, and yield amount (mass%) based on the amount of raw material used) are shown in Table 1.

### [Examples 2 to 6, Comparative Examples 1 to 9]

In step (B), 3-mercaptobutyric acid was produced in the same manner as in Example 1, except that concentrated sulfuric acid (98%) or concentrated hydrochloric acid was used in place of concentrated hydrochloric acid, and the conditions were changed to those shown in Table 1 or Table 2. The results are shown in Table 1 and Table 2.

**[Table 1]**

| | | Unit | Example 1 | Example 2 | Example 3 |
|---|---|---|---|---|---|
| Hydrolysis reaction | Type of acidic aqueous solution (hydrolysis) | - | Concentrated hydrochloric acid | Concentrated sulfuric acid | Concentrated sulfuric acid |
| | Acid concentration of acidic aqueous solution (hydrolysis) | mass% | 35 | 98 | 98 |
| | Amount of acidic aqueous solution (hydrolysis) | 9 | 60.5 | 11.5 | 6.0 |
| | Amount of acid | mol | 0.58 | 0.11 | 0.06 |
| | Equivalent of acid *1 (hydrolysis) | equivalent | 0.50 | 0.10 | 0.05 |
| | Amount of water (hydrolysis) *2 | g | 99.3 | 52.5 | 52.5 |
| | Amount of water (hydrolysis) *2 | mol | 5.51 | 2.91 | 2.91 |
| | Equivalent of water (hydrolysis) *1 | equivalent | 4.7 | 2.5 | 2.5 |
| | Ratio of water to acid | equivalent ratio | 9.5 | 25.4 | 48. 6 |
| | Reaction temperature | °C | 100 | 100 | 100 |
| | Reaction time | h | 3 | 3 | 4 |
| Post-treatment | Extraction solvent | - | Toluene | Toluene | Toluene |
| | Amount of extraction solvent | g | 80 | 80 | 80 |
| | Number of water washings | cycle | 0 | 0 | 0 |
| | Amount of water (water washing) | g | 0 | 0 | 0 |
| Reaction result | Yield amount | g | 117.4 | 125.6 | 125.1 |
| | Yield | % | 84 | 90 | 90 |
| | Yield amount/total raw material used | mass% | 26.0 | 31.8 | 32. 2 |

| | | | | | |
|---|---|---|---|---|---|
| *1: Equivalents relative to the thiocarboxylic acid ester *2: Total amount of water including water derived from acidic aqueous solution and additionally added water | | | | | |

**[Table 1] (continued)**

| | | Unit | Example 4 | Example 5 | Example 6 |
|---|---|---|---|---|---|
| Hydrolysis reaction | Type of acidic aqueous solution (hydrolysis) | | Concentrated sulfuric acid | Concentrated sulfuric acid | Concentrated sulfuric acid |
| | Acid concentration of acidic aqueous solution (hydrolysis) | mass% | 98 | 98 | 98 |
| | Amount of acidic aqueous solution (hydrolysis) | 9 | 6.0 | 34.5 | 58.0 |
| | Amount of acid | mol | 0.06 | 0.34 | 0.58 |
| | Equivalent of acid *1 (hydrolysis) | equivalent | 0.05 | 0.30 | 0.50 |
| | Amount of water (hydrolysis) *2 | g | 31.5 | 52.5 | 52.5 |
| | Amount of water (hydrolysis) *2 | mol | 1.75 | 2.91 | 2.91 |
| | Equivalent of water (hydrolysis) *1 | equivalent | 1.5 | 2.5 | 2.5 |
| | Ratio of water to acid | equivalent ratio | 29.2 | 8.5 | 5.0 |
| | Reaction temperature | °C | 100 | 100 | 100 |
| | Reaction time | h | 4 | 3 | 3 |
| Post-treatment | Extraction solvent | - | Toluene | Toluene | Toluene |
| | Amount of extraction solvent | g | 80 | 80 | 80 |
| | Number of water washings | cycle | 0 | 0 | 0 |
| | Amount of water (water washing) | g | 0 | 0 | 0 |
| Reaction result | Yield amount | g | 126.6 | 124.6 | 116.9 |
| | Yield | % | 91 | 89 | 84 |
| | Yield amount/total raw material used | mass% | 34.4 | 29.9 | 26.5 |

| | | | | | |
|---|---|---|---|---|---|
| *1: Equivalents relative to the thiocarboxylic acid ester *2: Total amount of water including water derived from acidic aqueous solution and additionally added water | | | | | |

**[Table 2]**

| | | Unit | Comparative Example 1 | Comparative Example 2 | Comparative Example 3 | Comparative Example 4 | Comparative Example 5 |
|---|---|---|---|---|---|---|---|
| Hydrolysis reaction | Type of acidic aqueous solution (hydrolysis) | - | Concentrated sulfuric acid | Concentrated sulfuric acid | Concentrated hydrochloric acid | Concentrated hydrochloric acid | Concentrated hydrochloric acid |
| | Acid concentration of acidic aqueous solution (hydrolysis) | mass% | 98 | 98 | 35 | 35 | 35 |
| | Amount of acidic aqueous solution (hydrolysis) | 9 | 1.0 | 2.5 | 96.5 | 121.5 | 243.0 |
| | Amount of acid | mol | 0.01 | 0.02 | 0.93 | 1.17 | 2.33 |
| | Equivalent of acid *1 (hydrolysis) | equivalent | 0.01 | 0.02 | 0.80 | 1.0 | 2.0 |
| | Amount of water (hydrolysis) *2 | 9 | 31.5 | 52.5 | 122.7 | 139.0 | 158.0 |
| | Amount of water (hydrolysis) *2 | mol | 1.7 | 2.9 | 6.8 | 7.7 | 8.8 |
| | Equivalent of water (hydrolysis) *1 | equivalent | 1.5 | 2.5 | 5.9 | 6.6 | 7.5 |
| | Ratio of water to acid | equivalent ratio | 174.9 | 116.6 | 7.4 | 6.6 | 3.8 |
| | Reaction temperature | °C | 100 | 100 | 100 | 100 | 100 |
| | Reaction time | h | 14 | 7 | 3 | 3 | 3 |
| Post-treatment | Extraction solvent | - | Toluene | Toluene | Toluene | Toluene | Toluene |
| | Amount of extraction solvent | 9 | 80 | 80 | 80 | 80 | 80 |
| | Number of water washings | cycle | 0 | 0 | 0 | 0 | 0 |
| | Amount of water (water washing) | 9 | 0 | 0 | 0 | 0 | 0 |
| Reaction result | Yield amount | g | 107.2 | 109.6 | 107.7 | 104.8 | 93.1 |
| | Yield | % | 77 | 79 | 77 | 75 | 67 |
| | Yield amount/total raw material used | mass% | 29.5 | 28.4 | 22.1 | 20.5 | 16.2 |

| | | | | | | | |
|---|---|---|---|---|---|---|---|
| *1: Equivalents relative to the thiocarboxylic acid ester *2: Total amount of water including water derived from acidic aqueous solution and additionally added water | | | | | | | |

**[Table 2] (continued)**

| | | Unit | Comparative Example 6 | Comparative Example 7 | Comparative Example 8 | Comparative Example 9 |
|---|---|---|---|---|---|---|
| Hydrolysis reaction | Type of acidic aqueous solution (hydrolysis) | - | Concentrated sulfuric acid | Concentrated sulfuric acid | Concentrated sulfuric acid | Concentrated sulfuric acid |
| | Acid concentration of acidic aqueous solution (hydrolysis) | mass% | 98 | 98 | 98 | 98 |
| | Amount of acidic aqueous solution (hydrolysis) | 9 | 116.0 | 232.0 | 116.0 | 116.0 |
| | Amount of acid | mol | 1.16 | 2.32 | 1.16 | 1.16 |
| | Equivalent of acid *1 (hydrolysis) | equivalent | 1.0 | 2.0 | 1.0 | 1.0 |
| | Amount of water (hydrolysis) *2 | 9 | 52.5 | 52.5 | 52.5 | 52.5 |
| | Amount of water (hydrolysis) *2 | mol | 2.9 | 2.9 | 2.9 | 2.9 |
| | Equivalent of water (hydrolysis) *1 | equivalent | 2.5 | 2.5 | 2.5 | 2.5 |
| | Ratio of water to acid | equivalent ratio | 2.5 | 1.3 | 2.5 | 2.5 |
| | Reaction temperature | °C | 100 | 100 | 100 | 100 |
| | Reaction time | h | 3 | 3 | 3 | 3 |
| Post-treatment | Extraction solvent | - | Toluene | Toluene | Toluene | Toluene |
| | Amount of extraction solvent | 9 | 80 | 80 | 80 | 320 |
| | Number of water washings | cycle | 0 | 0 | 1 | 0 |
| | Amount of water (water washing) | 9 | 0 | 0 | 50 | 0 |
| Reaction result | Yield amount | g | 103.3 | 94.1 | 79.5 | 103.3 |
| | Yield | % | 74 | 67 | 57 | 74 |
| | Yield amount/total raw material used | mass% | 20.7 | 15.3 | 14.5 | 14.0 |

| | | | | | | |
|---|---|---|---|---|---|---|
| *1: Equivalents relative to the thiocarboxylic acid ester *2: Total amount of water including water derived from acidic aqueous solution and additionally added water | | | | | | |

### [Comparative Examples 10 and 11]

### Step A

The thiocarboxylic acid ester was obtained in the same manner as in Example 1.

### Step B

The flask containing the thiocarboxylic acid ester as the reaction product obtained in step (A) was charged with sodium hydroxide (solid) and distilled water in the amounts shown in Table 3, and the resultant was subjected to a hydrolysis reaction under the reaction conditions shown in Table 3. After completion of the hydrolysis, neutralization was carried out with concentrated hydrochloric acid in the amount shown in Table 3. Next, the reaction mixture was extracted with toluene in the amount shown in Table 3. The organic phase (toluene layer) was then washed with distilled water, using the amount and number of washes indicated in Table 3. The organic phase was concentrated by reducing the pressure to 3 kPa with a diaphragm pump and heating at a bath temperature of 40 to 60°C to remove toluene and acetic acid by distillation. Subsequently, the residue was distilled under reduced pressure at 0.5 kPa using a vacuum pump, and 3-mercaptobutyric acid was collected as the distillate. The distillation temperature was 60 to 70°C. The results (yield amount, yield, and yield amount (mass%) based on the amount of raw material used) are shown in Table 3.

**[Table 3]**

| | | Unit | Comparative Example 10 | Comparative Example 11 |
|---|---|---|---|---|
| Hydrolysis reaction | Type of alkali | - | NaOH | NaOH |
| | Amount of NaOH (hydrolysis) | g | 153.5 | 153.5 |
| | | mol | 3.84 | 3.84 |
| | Equivalent of NaOH *1 (hydrolysis) | equivalent | 3.3 | 3.3 |
| | Amount of water (hydrolysis) | g | 200 | 200 |
| | | mol | 11.1 | 11.1 |
| | Reaction temperature | °C | 20 | 20 |
| | Reaction time | h | 0.2 | 0.2 |
| Neutralization | Amount of hydrochloric acid (neutralization) | 9 | 412 | 412 |
| | Equivalent of hydrochloric acid (neutralization) | equivalent | 3.4 | 0.1 |
| Post-treatment | Extraction solvent | - | Toluene | Toluene |
| | Amount of extraction solvent | g | 80 | 80 |
| | Number of water washings | cycle | 2 | 0 |
| | Amount of water (water washing) | g | 150 | 0 |
| Reaction result | Yield amount | g | 65.0 | 109.1 |
| | Yield | % | 47 | 78 |
| | Yield amount/total raw material used | mass% | 4.7 | 10.0 |

| | | | | |
|---|---|---|---|---|
| *1: Equivalents relative to the thiocarboxylic acid ester | | | | |

As shown in Table 1, in the examples in which the amount of the acid catalyst used during the reaction was from 0.025 equivalents to 0.68 equivalents relative to the thiocarboxylic acid ester, the yield of 3-mercaptobutyric acid was 84% or more in all cases. On the other hand, as shown in Table 2, in Comparative Examples 1 to 9 in which the equivalent of acid catalyst was less than 0.025 or greater than 0.68, the yield of 3-mercaptobutyric acid was less than 80% in all cases. Table 3 also shows that the yield was even lower when the base sodium hydroxide was used in the hydrolysis reaction in step B.

## Claims

1. A method for producing a 3-mercaptocarboxylic acid represented by formula (1), the method comprising the following step (A) and step (B):
step (A): adding a thiocarboxylic acid to the carbon-carbon unsaturated bond of an α,β-unsaturated carboxylic acid to obtain a thiocarboxylic acid ester; and
step (B):
adding water and an acid catalyst to the thiocarboxylic acid ester for a hydrolysis reaction, wherein the hydrolysis reaction is under a condition where the amount of the acid catalyst used in the reaction is from 0.025 equivalents to 0.68 equivalents relative to the thiocarboxylic acid ester, and the amount of water is 1.0 equivalent or more relative to the thiocarboxylic acid ester, 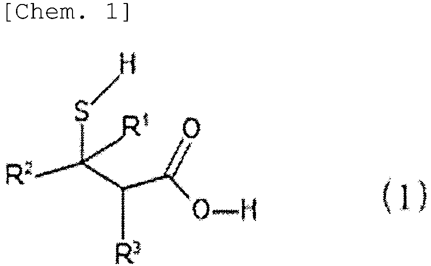 wherein R¹ to R³ each independently represent a hydrogen atom or an alkyl group having 1 to 5 carbon atoms.

2. The method for producing 3-mercaptocarboxylic acid according to claim 1, wherein in the step (B), the amount of water is from 1.0 equivalent to 5.5 equivalents relative to the thiocarboxylic acid ester.

3. The method for producing 3-mercaptocarboxylic acid according to claim 1 or 2, wherein the addition reaction in the step (A) is carried out in the presence of a solvent, and after the reaction, the solvent is distilled off, followed by the step (B).

4. The method for producing 3-mercaptocarboxylic acid according to claim 1 or 2, wherein the acid catalyst in the step (B) is hydrochloric acid or sulfuric acid.

5. The method for producing 3-mercaptocarboxylic acid according to claim 1 or 2, wherein the thiocarboxylic acid is thioacetic acid.

6. The method for producing 3-mercaptocarboxylic acid according to claim 1 or 2, wherein the α,β-unsaturated carboxylic acid is crotonic acid.

7. The method for producing 3-mercaptocarboxylic acid according to claim 1 or 2, wherein the acid catalyst is sulfuric acid, the thiocarboxylic acid is thioacetic acid, the α,β-unsaturated carboxylic acid is crotonic acid, and the 3-mercaptocarboxylic acid represented by formula (1) is 3-mercaptobutyric acid.
